(19) Europäisches Patentamt

European Patent Office

Office européen des brevets

(11) **EP 1 293 557 B1**

(12) **EUROPEAN PATENT SPECIFICATION**

(45) Date of publication and mention
of the grant of the patent:
**21.06.2006 Bulletin 2006/25**

(51) Int Cl.:
*C11D 17/04* (2006.01)   *C11D 17/00* (2006.01)
*A61K 8/03* (2006.01)   *A61K 8/06* (2006.01)
*A61K 8/11* (2006.01)   *A61Q 5/02* (2006.01)

(21) Application number: **02255110.5**

(22) Date of filing: **22.07.2002**

(54) **Water-soluble package containing a fluid composition with a visually discrete capsule for emulsion or dispersion layer**

Wasserlösliche Packung enthaltend eine flüssige Zusammensetzung mit einer visuell unterscheidbaren Kapsel oder Emulsion oder Dispersionsschicht

Emballages solubles dans l'eau contenant une composition liquide avec des capsules, ou une émulsion ou une couche de dispersion discernable visuellement

(84) Designated Contracting States:
**AT BE BG CH CY CZ DE DK EE ES FI FR GB GR IE IT LI LU MC NL PT SE SK TR**

(30) Priority: **28.08.2001 US 941219**

(43) Date of publication of application:
**19.03.2003 Bulletin 2003/12**

(73) Proprietors:
• **UNILEVER PLC**
**London EC4P 4BQ (GB)**
Designated Contracting States:
**CY GB IE**
• **UNILEVER N.V.**
**3013 AL Rotterdam (NL)**
Designated Contracting States:
**AT BE BG CH CZ DE DK EE ES FI FR GR IT LI LU MC NL PT SE SK TR**

(72) Inventors:
• **Hsu, Feng-Lung Gordon,**
**Edgewater,**
**New Jersey 07020 (US)**
• **Lee, Kwang Ho,**
**c/o Unilever Research. U.S. Inc.**
**Edgewater,**
**New Jersey 07020 (US)**

(74) Representative: **Elliott, Peter William et al**
**Unilever Patent Group**
**Colworth House**
**Sharnbrook**
**Bedford, MK44 1LQ (GB)**

(56) References cited:
**WO-A-02/057402**   **GB-A- 2 254 857**
**US-A- 4 348 292**

**Description**

**FIELD OF THE INVENTION**

Water-soluble package containing a fluid composition with a visually discrete hydrophobic layer in the form of emulsion or capsules or dispersion.

**BACKGROUND OF THE INVENTION**

Detergent compositions and personal care compositions are provided in many forms. Probably the most prevalent forms are granular and liquid compositions. More recently unit dose forms of detergent have been proposed in the form of compressed tablets of detergent powder or water-soluble packages. The unit dose forms are preferred by some consumers, in that the dose is pre-measured and, consequently, the unit dose form is faster, easier and less messy to use. Water-soluble packages filled with liquid detergent composition are desired especially by consumers who are used to liquid detergents.

Water-soluble unit dose liquid detergent packages are known. See for instance Kennedy (US Patent 4,973,416), Dickler et al. (US Patent 6,037,319), Haq (US Patent 4,416,791) and Richardson (US Patent 4,115,292).

In many articles of commerce, particularly consumer products, it is desirable to separate certain ingredients, yet have them disposed in a common container. Separation is particularly beneficial where one or more ingredients have negative interactions with each other. For example, in laundry detergents, enzymes are useful in removing stains but it is also best to separate them from other constituents, such as sources of alkalinity and surfactants, especially anionic surfactants like linear alkylbenzene sulfonates or alkyl sulfates. Bleaches, vitamins, perfumes, vegetable oils, plant extracts and ceramides are further examples of ingredients that sometimes need to be separated from the rest of the detergent or personal care composition.

A known technique for separating ingredients in a common container includes encapsulation. Encapsulation technology is well known for different applications. Generally, encapsulation includes a medium that surrounds at least one component and thereby provides a barrier between the "encapsulated" component and other components. The barrier is typically temporary and is designed to break down and release the encapsulated material at a desired time, such as at a particular temperature, upon reaction or dissolution with chemicals, or due to mechanical stress. Methods of encapsulation include coacervation, liposome formation, granulation, coating, emulsification, atomization and spraycooling.

See, for instance, the disclosures of enzyme encapsulates and encapsulation processes: Falholt et al. (U.S. Patent 4,906,396, UK 2,186 884, and EP 0 273 775), Tsaur et al. (U.S. Patents 5,434,069 and 5,441,660), Ratuiste et al. (U.S. Patent 5,589,370). JP 41003667 discloses a dialysis of a protein solution against polyol-base polymer.

See also Mitchnik et al. (U.S. Patent 5,733,531) and Leong (U.S. Patent 5,296,166). WO 01/05949 discloses a method for increasing the density of enzyme capsules.

In traditionally packaged products (e.g., bottles or tubes), capsules typically need to be dispersed uniformly throughout the product, to provide uniform dispensing from the package. Such is not the case with unit dose forms of detergents: although uniform dispersion is possible, it is not necessary, since the whole package is used in a single application.

It is desirable to increase the visual appeal of the package and also provide a unique appearance to be associated by consumers with a particular product. In addition, it is desirable to provide a visual signal to a consumer of the presence of special (i.e., benefit) ingredient in the composition. At the same time, the benefit ingredients, e.g. enzymes or bleaches or moisturising oils, need to be protected to preserve their activity in the composition, especially when such composition includes water and/or surfactant.

Thus, it is desirable to provide a water-soluble unit dose package containing a liquid composition, which contains a visually discrete, hydrophobic (so, immiscible with the rest of the composition) layer. The layer serves to provide a unique appearance to the product, and may, at the same time, be employed to entrap, or protect, a benefit agent and/or a colorant.

**SUMMARY OF THE INVENTION**

The present invention includes a water-soluble package for use in a single application according to claim 1

Preferred fluid compositions are detergent or personal care compositions.

The following detailed description and the examples illustrate some of the effects of the inventive compositions. The invention and the claims, however, are not limited to the following description and examples.

## DETAILED DESCRIPTION OF THE INVENTION

Except in the operating and comparative examples, or where otherwise explicitly indicated, all numbers in this description indicating amounts of material or conditions of reaction, physical properties of materials and/or use are to be understood as modified by the word "about." All amounts are by weight, unless otherwise specified.

For the avoidance of doubt the word "comprising" is intended to mean including but not necessarily "consisting of" or "composed of." In other words the listed steps or options need not be exhaustive.

The term "continuous" does not necessarily mean "isotropic". The term "continuous" is used herein to denote the phase which is predominant in volume during emulsification or dispersion of discontinuous phase in the continuous phase.

The term "hydrocarbon oil" as used herein means a hydrocarbon oil having a maximum viscosity of about 10 kg/(m)(sec), preferably no greater than about 5 kg/(m)(sec).

The term "wax" as used herein means a hydrophobic material which is a solid at 20°C. By "solid" is meant the ingredient is not mobile at 20°C.

The term "fluid" means that the composition is mobile at 20°C.

## LAYER COMPOSITION

The layer composition according to the present invention is immiscible with the rest of the composition. Generally, the layer composition is hydrophobic in nature, to prevent its diffusion into the rest of the composition, the rest of the composition typically being an isotropic composition containing at least some water.

The hydrophobic ingredient for the layer composition is generally selected from the group consisting of paraffin, wax, oil, petrolatum, a hydrophobic polymer and mixtures thereof.

In one embodiment of the invention, the hydrophobic layer is employed in combination with a hydrophobic benefit agent and/or colorant (e.g. oil-soluble colorant). In another embodiment of the invention, a hydrophobic ingredient forms a continuous phase (also sometimes referred to herein as "shell") which surrounds a discontinuous phase. The layer composition is in the form of capsules or an emulsion or a dispersion. The discontinuous phase may itself be a benefit agent and/or a colorant or it may contain an additional benefit agent and/or colorant.

Capsules have traditionally been required to prepare a uniform dispersion of a benefit agent throughout the packaged composition (e.g., bottle) to ensure consistent dispensing of the encapsulated ingredient each time the bottled composition is used. In the present invention, the whole package is used at once, in a single application, and the formulation contains a layer rather than a uniform distribution of capsules throughout the fluid composition. Consequently, although it is possible to employ capsules (as long as they are positioned in the layer), it is not necessary to do so. Indeed, in the preferred embodiment of the invention, a layer is selected from a single phase hydrophobic solution (thus, forming an in-situ oil-in-water emulsion in the aqueous unit dose composition), an emulsion (water-in-oil emulsion which becomes water-in-oil-in water emulsion in the aqueous unit dose composition) or a dispersion, most preferably a hydrophobic solution or an emulsion, to avoid the unnecessary complexity and cost of capsule preparation. In the most preferred embodiment, the layer is an emulsion comprising a hydrophobic continuous phase and an aqueous discontinuous phase (water-in-oil emulsion which becomes water-in-oil-in water emulsion in the aqueous unit dose composition).

The fluid composition within the package includes from 0.1 to 50% of the layer composition, more preferably from 0.5 to 35%, most preferably from 0.7 to 10%, and optimally from 1% to 2%, in order to provide sufficient visual appeal and/or delivery of the layer ingredient (% by weight of the total fluid composition).

### Hydrophobic Ingredient

In one preferred embodiment of the invention, a mixture of a thermoplastic block co-polymer and a hydrocarbon oil is employed, particularly when it is desired to make a transparent/translucent (colored or uncolored) layer or the continuous phase. The block co-polymers particularly suitable in the present invention are block co-polymers containing at least one rigid block and at least one flexible block. The mixture of the hydrocarbon oil and the block co-polymer according to the present invention is isotropic at 20oC. It should be understood that since the co-polymer is not pourable at 20oC (indeed, it is solid), it may be difficult to combine the co-polymer with the oil at such temperature to ascertain whether the mixture is isotropic. According to the present invention, a mixture may be formed at any suitable temperature at which the liquefied co-polymer forms an isotropic liquid mixture with the oil. The copolymer/oil mixtures suitable for use in the present invention, however, remain isotropic after cooling. Suitable isotropic mixtures have transmittance of at least 50%, preferably at least 70%, as measured by UV-visible spectrophotometer (measured in visible light range).

**Block Co-polymer**

In one embodiment of the invention, the co-polymer employed in the layer is selected from the group consisting of a triblock co-polymer, radial co-polymer, and multiblock copolymer, the co-polymer comprising at least one triblock with a structure: rigid block---flexible block---rigid block. In another embodiment of the invention, a di-block co-polymer is employed; rigid block-flexible block. Preferably the rigid block is styrene-type polymer, and the flexible block is rubber-type polymer. By virtue of employing the co-polymer, the viscosity of the oil is increased, and the viscous or hardened layer or continuous phase is formed, yet the resulting composition is sufficiently soft and friable to release the discontinuous phase or the benefit ingredient in normal use. The copolymer blends uniformly with oil at a temperature which is much lower than the melting point of wax, thus allowing for protection of temperature-sensitive ingredients, e.g. bleach, perfume, enzyme, vegetable oil, etc. A further advantage of using the co-polymer is that it is not necessary (although possible) to use a surfactant in preparing a uniform distribution of the discontinuous phase in the continuous phase; the avoidance of the surfactant makes the encapsulation process easier and cheaper. Furthermore, the absence of surfactant improves the stability of the encapsulated ingredient, since the surfactant provides for a potential channel of penetration for an external environment.

The preferred co-polymers are transparent and uncoloured, in order to attain a transparent and uncoloured continuous phase.

Examples of suitable co-polymers include but are not limited to those that are described in Morrison et al. (U.S. Patent 5, 679, 694).

Each of the diblock, triblock, radial block and/or multiblock copolymers in the invention contains at least two thermodynamically incompatible segments. By the expression thermodynamically incompatible with respect to the polymers, it is meant that the polymer contains at least two incompatible segments, for example at least one hard and one soft segment. In general, in a triblock polymer, the ratio of segments is one hard, one soft, one hard or an A-B-A copolymer. The multiblock and radial block copolymers can contain any combination of hard and soft segments, provided that there are both hard and soft characteristics. In the diblock copolymer, the blocks are sequential with respect to hard and soft segments.

Commercially available thermoplastic rubber type polymers which are especially useful in forming the compositions of the present invention are sold under the trademark Kraton® by Shell Chemical Company. The Kraton® rubber polymers are described as elastomers which have an unusual combination of high strength and low viscosity and a unique molecular structure of linear diblock, triblock and radial copolymers. Each molecule of the Kraton® rubber is said to consist of block segments of styrene monomer units and rubber monomer and/or comonomer units. Each block segment may consist of 100 or more monomer or comonomer units. The most common structure is the linear ABA block type; styrene-butadienestyrene (SBS) and styrene-isoprene-styrene (SIS), which is the Kraton® D rubber series.

A second generation polymer of this general type is the Kraton® G series. This copolymer comprises a styrene-ethylene-butylene-styrene type (S-EB-S) structure. The Kraton® G series is preferred in the practice of the invention, as the copolymers of this series are hydrogenated and thus more thermally stable; that is, decomposition is less likely to occur during blending of the G series polymers with the oil (the D series polymers having unsaturation within the rubber block). The Kraton® G rubbers are indicated as being compatible with paraffinic and naphthenic oils and the triblock copolymers are reported as taking up more than 20 times their weight in oil to make a product which can vary in consistency from a "Jello® " to a strong elastic rubbery material depending on the grade and concentration of the rubber. The diblock polymers include the AB type such as styrene-ethylenepropylene (S-EP) and styrene-ethylenebutylene (S-EB), styrene-butadiene (SB) and styrene-isoprene (SI).

Preferably, when Kraton® series block co-polymers are employed (i.e., styrene-elastomer block co-polymers), the oil is essentially free of silicone-containing oils, in order to obtain optimum isotropic mixtures. By "essentially free" is meant that in the Kraton® /oil continuous phase, the amount of silicone-containing oil is preferably less than 2%, by weight of the continuous phase, more preferably less than 1%, most preferably less than 0.5% and optimally is 0%.

The preferred polymer is the diblock co-polymer (having rigid-flexible blocks), even in the absence of a triblock or radial co-polymer. Kraton® 1702 is a diblock co-polymer (styrene-ethylene/propylene). The properties of Kraton® 1702 make it more suitable for use as a viscosity modifier in making the emulsion, rather than capsules. Kraton® 1702 is particularly preferred when a transparent layer or a transparent hydrophobic continuous phase is desired.

In another embodiment of the invention, a triblock copolymer of the Kraton® G type, in particular Kraton® G-1650. Kraton® G-1650 is an SEBS triblock copolymer which has a specific gravity of about 0.91, and is said to have a tensile strength of about 3.45 newton/m2 as measured by ASTM method D-412-tensile jaw tester separation speed 25.4 cm/min. The styrene to rubber content of Kraton® G-1650 is said by the manufacturer to be about 29:71, and the Brookfield viscosity is about 8 kg/(m)(sec)(toluene solution, at 25°C, 25%w). The Shore A hardness is about 75.

If the transparent capsules (rather than merely an emulsion) are desired, preferably a mixture of Kraton® 1650 with Kraton® 1702 is employed, even though Kraton® 1650 is sufficient on its own. The mixture may be preferred in some cases, in order to increase the friability of the capsules, while preserving transparency. When using the mixture of two Kraton polymers, the weight ratio of Kraton® 1650 to Kraton® 1702 is generally from 1:10 to 10:1, more preferably from 3:1 to 7:1, most preferably from 2:1 to 5:1, and optimally from 1:1 to 4:1.

The block co-polymer is employed in the inventive compositions generally in an amount of from 0.1% to 15%, more preferably from 0.5% to 10%, most preferably from 0.5% to 7%, and optimally from 1% to 4%, by weight of the continuous phase (or by weight of the layer, if only a hydrophobic phase is present).

In another preferred embodiment, a mixture of oil and wax is employed to produce an oil/wax dispersion with a viscosity at shear rate of $10^{-4}$ l/sec in the range of 10kg/(m)(sec) to 5000 kg/(m)(sec). Suitable commercial oil/wax mixtures include Petrolatum® and Tro Grees®.

In yet another embodiment, a mixture of oil, wax, and the block co-polymer is employed. In general, the capsules or emulsions which employ a mixture of oil, wax and the block co-polymer exhibit better transparency and maintain friability for release. In such mixtures, the block copolymer is present generally in an amount of from 0.1 to 10%, more preferably from 0.5% to 7%, and most preferably from 1% to 5%. The wax is present generally in an amount of from 0.1% to 30%, more preferably from 0.5% to 15%, and most preferably from 1% to 7%.

**Hydrocarbon Oil**

Natural or synthetic hydrocarbon oil or mixtures thereof may be employed. Generally, the hydrocarbon oil may be a paraffinic oil, a naphthenic oil, natural mineral oil or the like. Examples include but are not limited to mineral oil, castor oil, vegetable oil, corn oil, peanut oil, jojoba oil, 2-ethylhexyl oxystearate (and other alkyl oxystearates), acetylated lanolin alcohol, alkyl palmitates such as isopropyl palmitate, 2-ethylhexyl palmitate, glycerol triacetates, disopropyl adipate, dioctyl adipate (and other alkyl adipates), isopropyl myristate, C12 to C15 alcohol benzoates, and the like.

Most preferably, the oil is mineral oil, because it is both economic and most easily processable.

Preferably, when Kraton® series block co-polymers are employed (i.e., styrene-elastomer block co-polymers), the oil is essentially free of silicone-containing oils, in order to obtain optimum isotropic mixtures. By "essentially free" is meant that in the Kraton® /oil continuous phase, the amount of silicone oil is preferably less than 2%, by weight of the continuous phase, more preferably less than 1%, most preferably less than 0.5% and optimally is 0%.

The preferred oils are transparent and uncoloured, in order to attain a transparent and uncoloured continuous phase (although coloured continuous phase is also included within the scope of the invention).

When the layer is in the form of capsules or an emulsion or a dispersion, the hydrophobic continuous phase may include a surfactant as an emulsifier. Suitable surfactants are low HLB surfactants, which may be anionic, cationic, amphoteric, and nonionic, preferably having an HLB of 1 to 10, more preferably from 2 to 7 and most preferably less than 5. In the most preferred embodiment, the surfactant is Neodol® 25-3 available from Shell Chemical Co. The continuous phase generally includes from 0 to 10% of a surfactant, more preferably from 0.1 to 5%, most preferably from 0.3 to 4%, and optimally from 0.5% to 3%, in order to form an emulsion, yet to avoid the formation of a reverse emulsion (% by weight of the total continuous phase).

**Discontinuous Phase**

The desired ingredient to be protected (e.g., benefit ingredient or a colorant) may form a continuous phase with the hydrophobic ingredient (it can then be co-melted with the hydrophobic material) or it may form a discontinuous (hydrophilic or incompatible hydrophobe) phase. In the latter case, the hydrophobic material forms a continuous phase, which surrounds a discontinuous phase. A hybrid of the two cases is also possible, i.e. both the continuous and discontinuous phases contain benefit ingredient(s) and/ or colorant (s).

If present, the discontinuous phase of the inventive capsules is itself and/or comprises a benefit agent and/or a colorant. In some embodiments of the invention, the discontinuous phase is itself a benefit agent, e.g. a vegetable oil, such as sunflower seed oil, in personal care compositions. In other embodiments, the discontinuous phase is itself a colorant (e.g. a solid pigment). Still in other embodiments the discontinuous phase serves as a vehicle for a benefit agent and/or colorant. And still in other embodiments of the invention the discontinuous phase may itself be a benefit agent and/or colorant and also further include an additional benefit agent and/or colorant. According to the present invention, the discontinuous phase is immiscible with the continuous phase, to prevent the exposure of the continuous phase to the environment outside the capsule.

The discontinuous phase may be a solution (aqueous or oil), an oil, an emulsion, a dispersion, or a solid. The preferred form of the discontinuous phase is an oil or a solution (oil or aqueous solution), due to the relative ease of incorporation of the oil or the solution into the continuous phase. The layer may include more than one discontinuous phase.

If the additional benefit agent/colorant is oil-soluble, than an oil is chosen to carry the benefit agent/colorant in the discontinuous phase; if the benefit agent/colorant is water-soluble, than the discontinuous phase is an aqueous solution. Of course, as mentioned above, solids may be employed, without making a solution.

The discontinuous phase may be present in an amount of from 0.01 to 45%, more preferably from 5 to 45%, most preferably from 10 to 40%, and optimally from 20 to 35%, (% by volume of the capsule or the emulsion or the dispersion) in order to deliver sufficient benefit agent/colorant, provide an adequate protection for the benefit agent/colorant and to maintain the ease of processing.

For capsules/emulsions/dispersions which contain a discontinuous phase, the continuous phase may sometimes be referred to hereinafter as a "shell" or "shell material".

**Benefit Agent**

For simplicity, the material entrapped within the shell, either directly, or as a discontinuous phase, will be referred to as an "enzyme". However, it is within the scope of the present disclosure that materials other than enzymes can be encapsulated by the techniques disclosed herein. The choice of the benefit agent depends largely on whether the final consumer composition is a detergent composition or a personal care composition. As mentioned above, the continuous or discontinuous phase itself may represent a benefit agent, so it is not necessary that an additional benefit agent be present. Thus, an additional benefit agent may be present in an amount of from 0 to 100%, preferably 0.01 to 50%, more preferably 0.1 to 20%, by weight of the discontinuous phase.

Typical benefit agents include, but are not limited to a bleach, a bleach precursor, a surfactant, an enzyme, a whitening agent, a fabric softener, an anti-wrinkle compound, a dye fixative, dye transfer inhibitors, anti-redeposition polymers, soil release polymers, an anti-foam agent, a perfume, a silicone oil, a vegetable oil, a vitamin, a plant extract, a hydroxy acid, an anti-oxidant, an anti-bacterial agent, a moisturizer, and mixtures thereof.

If the encapsulated material is an enzyme, the preferred enzymes include proteases, lipases, cellulase, amylase, bleaching enzymes and the like. When selecting enzymes for a liquid detergent system, the most preferred enzymes include proteases and cellulases.

In the case of an enzyme, the discontinuous phase is an aqueous solution of the enzyme. The aqueous enzyme solution may optionally contain a low HLB surfactant, in order to further enhance the formation of the emulsion. If present, the surfactant may be chosen from and employed in the same amounts as the surfactants described above for the continuous phase. The level of the surfactant can be reduced or even eliminated, particularly if suitable agitation is used. Furthermore, the need for surfactant is entirely eliminated if the shell material is a mixture of thermoplastic polymer with oil, rather than a wax/oil mixture.

**Colorant**

The colorant may be a dye or a pigment. Dyes are preferable, since they are water-soluble and thus are more easily incorporated into the layer emulsion, compared to pigments which are typically not water-soluble. Most preferably, a water-soluble dye is entrapped, alone or in the mixture with a benefit agent, within a transparent, uncoloured continuous phase.

Most preferably, the layer is an emulsion or dispersion containing both the benefit agent and the colorant, within a transparent continuous phase, to provide a visual signal to the consumer that a composition contains an additional beneficial ingredient.

The emulsion/dispersion may be prepared by any known method, but preferably the emulsion or dispersion is prepared by mixing the continuous and discontinuous phases, the latter being or containing the ingredient to be encapsulated, e.g. bleach solution or a vegetable oil. In the preferred embodiment, the co-polymer is melted, mixed with oil, then the discontinuous phase is added, with stirring (agitation), to ensure uniform mixing of the ingredients. The resulting emulsion/dispersion is preferably kept at a temperature in the range from 40°C to 95°C. Most preferably, the use of direct heat is avoided. A most preferred temperature range is from 60°C to 75°C.

**POSITION OF THE LAYER**

Position of the layer is defined by the relative densities of the layer composition and the rest of the fluid composition. Most liquid detergents or liquid personal care compositions have a density of 1 or slightly below or above, i.e. in the range of from 0.9-1.1 g/L. If the hydrophobic layer has the density above that of the of the rest of the composition, it positions itself at the bottom of the water soluble package; if its density is lower than the density of the rest of the composition, it positions itself at the top of the package. If the densities of the layer and the rest of the composition match, the layer positions itself at any intermediate position between the top and the bottom. The stable intermediate position is difficult, if at all possible to achieve, since the layer is likely to break up.

The intermediate position, however, is possible, even theoretically, only if the density difference is less than 0.001g/L. Thus, the intermediate position is both unlikely and not preferred.

For instance, enzyme emulsion or capsules typically have a lower density than a liquid composition for which they are intended, especially liquid detergent composition. In this case, the density of the enzyme capsule/emulsion is less than 1, as a result of the density of the hydrophobic ingredient ranging from 0.8 to 0.9 g/L. Since and the density of the enzyme capsule/emulsion is less than 1, the enzyme capsules will float and form a layer on the top portion of the liquid formulation.

## LAYER AT THE BOTTOM

To increase the density of the layer, it is necessary to add an ingredient with density greater than 1g/L, preferably greater than 1.05 g/L, and most preferably greater than 1.1 g/L to the layer composition. Such ingredients may be solid or liquids. Suitable high density solid ingredients include polyols (e.g., sugar, sorbitol, glycerine, alkene (e.g., ethylene, propylene) glycols, hydrophobically treated silica, zeolite, titanium dioxide, inorganic salt.

Hydrophobic particles, such as hydrophobically treated silica, may be mixed with hydrophobic continuous phase prior to the addition of the discontinuous phase (if the discontinuous phase is present). Hydrophilic particles, such as color pigment, are preferably added to hydrophilic phase prior to its addition to the continuous phase for preparation of an emulsion.

When the concentration of solute is higher than its saturation concentration, a portion of solute will be crystallized out as solid particles. These particles are functional as hydrophilic particles in hydrophilic phase. Sugar base type of solute, especially sorbitol, is most preferable, since it does not have a tendency of salting out the benefit ingredients in hydrophilic phase. Furthermore, the viscosity of hydrophilic phase is increased with the higher concentration of sugar base type of solute in a hydrophilic phase. The higher viscosity of a discontinuous phase improves the stability of emulsion.

When there is an excess of the solute, in order to keep the solid particles suspended, it is highly desirable to increase the viscosity of a suspending phase. Preferred compositions, however, incorporate a heavy liquid, to obviate the need for suspending the particles. Suitable heavy liquid include, but are not limited to some silicones and halogenated oils. Dow 593 Silicon Fluid has a density of about 1.02g/L at 25°C and can be incorporated in a hydrophobic continuous phase. The density of halogenated oils in general is greater than 1g/L and they may be also incorporated into a hydrophobic continuous phase.

The amount of a high density ingredient in the layer composition depends on several factors, such as density and fraction of each component of the fluid composition. The amount is generally determined from the principle that the density of fluid composition should be less than the sum of densities of components in the layer. The following equation sets out the relationship between the density of the layer and the density of individual components and their fraction in the layer:

$$\rho_{layer} = \sum_{i=1}^{n} (\rho_i) \times (x_i)$$

wherein i is component i;
n is total number of components;
$\rho_i$ is the density of component i;
$x_i$ is the fraction of component i (fraction of its weight relative to the weight of the layer).

## FLUID COMPOSITION

The fluid composition contained within the package may be a liquid, a gel or a paste. If the substance is a liquid then preferably the liquid has a viscosity between 0.1 and 1 kg/(m)(sec), more preferably between 0.3 and 8 kg/(m)(sec), even more preferably between 0.5 and 0.7 kg/(m)(sec), and most preferably about 0.6 kg/(m)(sec), when measured at 20°C at 10s-1. In a preferred embodiment of the invention the composition is present in an amount of between 10 and 500ml, preferably between 20 and 100ml, most preferably between 25 and 50ml. Suitably, the package contains between 20 and 30ml of a fluid composition.

Various detergent compositions include, but are not limited to laundry compositions, hard surface cleaners, dishwashing compositions. In a particularly preferred embodiment of the invention the fluid composition is a laundry treatment agent such as a laundry detergent, fabric conditioner or fabric care formulation.

Preferred laundry compositions comprise, in addition to the inventive capsules, a surfactant, in an amount from 1 to 70% more preferably from 10 to 50%, most preferably from 15 to 35%, and optimally from 17 to 30% (% by weight of the laundry composition). Suitable detergent and laundry surfactants are well known to one of ordinary skill in the art and may in general be chosen from anionic, nonionic, amphoteric, and cationic surfactants. Preferably, the surfactant in the laundry compositions is anionic and/or nonionic, especially linear alkylbenzene sulfonate, alkyl ether sulfate, especially, alcohol ethoxylates and mixtures thereof.

In addition to the surfactant and the layer composition, the preferred laundry composition may include one or more well-known laundry ingredients, such as builders (from 0.1 to 40% for powders, from 0.1 to 20% for liquids), anti-redeposition agents, fluorescent dyes, perfumes, soil-release polymers, colorant, enzymes, etc.

Preferred detergent compositions according to the invention contain a layer in the form of emulsion or capsules with encapsulated bleach or bleach system, preferably in solution. Any bleach suitable for detergent application may be included. Examples include, but are not limited to chlorine bleaches, peracids, bleach precursors, alone or with oxygen sources.

In the layer, if a bleach or an enzyme is entrapped, the layer would generally include from 10% to 60%, more preferably from 15% to 50%, most preferably from 20% to 45% and optimally from 25% to 40%, of a bleach, in order to deliver an optimum benefit for minimum cost (% by weight of the layer).

Personal care compositions according to the present include products which are rinsed off after application (e.g., shower gels, shampoos) and products that are left on after application (e.g., cosmetic lotions, gels and creams). Various personal care compositions include, but are not limited to, facial or body cleansing compositions, shampoo compositions, conditioner compositions, and cosmetic compositions. Personal care compositions may be in the form of solution, lotion, cream, or gel, and any combinations thereof.

Preferred personal care compositions comprise, in addition to the layer composition, a cosmetically acceptable vehicle, in an amount from 0.1 to 70%, more preferably from 3 to 85%, most preferably from 5 to 95% and optimally from 10 to 99% (% by weight of the composition). Suitable vehicles are well known to one of ordinary skill in the art and may in general be chosen from isotropic liquid formulas or structured liquid formulas. Preferably, the vehicle in the personal care compositions is structured liquid formulas especially lamellar forming (structured) liquid formulas. In addition to the vehicle and the layer composition, the preferred personal care composition may include one or more well-known personal care ingredients, such as viscosity builders (from 0.1 to 30%), pH controllers (stabilizers) (from 0.005 to 20%).

Preferred personal care compositions are personal wash or shampoo or hair conditioning compositions, wherein the layer contains a combination of a benefit agent and a colorant, with the benefit agent chosen from vitamins, anti-bacterial agents, vegetable oils, cationic surfactant (e.g. quaternary ammonium) and mixtures thereof.

Vitamins include, but are not limited to A, E, C. Anti-bacterial agents include, but are not limited to Triclosan. Vegetable oils include but are not limited to sunflower seed oil. The personal wash compositions include, in addition to the capsules and the vehicle, a surfactant, especially Tegobetaine (Cocamidopropyl Betaine). The surfactant is included generally in an amount, more preferably form .1 to 10%, most preferably form 1 to 30%, and optimally from 20 to 60% (% by weight of the total composition).

The fluid compositions include some water, typically 1 to 15% water.

Preferably, the detergent or personal care composition is a transparent composition containing transparent or colored layer, and packaged in the clear/transparent package.

## WATER-SOLUBLE BODY PORTION

The package is preferably made of a clear, heat sealable, cold water soluble film such as polyvinyl alcohol. Thickness could range from 25 to 100 $\mu$m, more preferably from 35 to 80 $\mu$m, most preferably from 45 to 55 $\mu$m. Other materials from which the package can be made include but are not limited to methyl hydroxy propyl cellulose.

The water soluble film, at least of the body wall, is thermoformable and, in one embodiment of the invention, is polyvinyl alcohol, or a polyvinyl alcohol derivative. Preferably the water soluble film of the base wall is the same material as that used to make the body wall. It is preferred that the body wall be thermoformed rather than cold formed because cold forming stresses the film and weakens the end package as a result.

The packages of the invention may be prepared from polyvinyl alcohol film, or other suitable material, which is filled, then sealed. Preferably, a thermoforming process is employed.

The package may take many shapes as viewed in a plan view, such as rectangular, square, round, triangular, etc.

In use, the package is mixed with water (e.g., inside a laundry machine or a dishwasher), or applied to the body and water is added, simultaneously or consecutively, in order to release the contents of the package.

The following specific examples further illustrate the invention, but the invention is not limited thereto. Suppliers and chemical description of the ingredients used in the examples are summarized in the following table:

| Trade Name (if appropriate) | Chemical Name | Supplier |
| --- | --- | --- |
| Bowax® 800 | Petroleum, Hydrocarbon, Microcrystalline Wax | IGI |

Table continued

| Trade Name (if appropriate) | Chemical Name | Supplier |
|---|---|---|
| Kraton® 1702 | Styrene-(Ethylene-Propylene) di-block co-polymer | Shell |
| Sorbitol | D-Glucitol | SPI Polyols |
| Petrolatum | Hydrocarbons | Penreco |
| | Titanium Oxide | Dupont |
| | Cationic surfactant: Dihydrogenated Tallow Dimethyl Ammonium Chloride | Akzo Nobel |
| Tergitol® 15 S-9 | Nonionic surfactant: Alkyloxypolyethyleneoxyethanol | Union Carbide |
| Silwet® L-77 | Polyalkylene oxide modified hertamethyltrisiloxane 84%, and Allyloxypolyethylene glycol methyl ether 16% | OSI Specialtie s |
| Mineral Oil | Hydrocarbons | Fischer Scientific |
| Properase® 1600 L | Protease | Genencor Internatio nal, Inc |

EXAMPLE 1

This example demonstrates the manipulation of capsule density.
An emulsion was made as follows:

| Petrolatum | 200 g |
|---|---|
| Titanium oxide | 2 g |
| dihydrogenated tallow dimethyl ammonium chloride | 20 g |
| 70% Sorbitol aqueous solution | 140 g |

Petrolatum was heated to 60°C, followed by the addition of titanium oxide for adding white color. Subsequently, the temperature was raised to 65.6°C. After the addition of the cationic surfactant, the temperature dropped. The temperature was raised again to 65.6°C, then sorbitol solution was added. Upon addition of sorbitol, temperature dropped to 46.1°C. Temperature was allowed to climb back to 68.3°C.
After mixing for 45 minutes, the emulsion was added dropwise into curing solution containing:

| Water | 950 g |
|---|---|
| Tergitol® 15-S-9 | 50 g |

Capsules made from this emulsion did not sink. Additional 15g 70% sorbitol was added to the emulsion and re-tested. The capsules still floated. Another additional 25g of 70% sorbitol solution was added, thus bringing the total sorbitol level to 180g. When capsules were dropped into curing solution, they initially began to sink, followed by rising to the surface, then sank to the bottom and stayed. Additional 20g of sorbitol was added thus bringing the total sorbitol level to 200g. Capsules of this final emulsion sank to the bottom and never floated to the surface. The quality of the capsules was the same, but the high density capsules were easier to process.

EXAMPLE 2

Capsules within the scope of the invention were prepared. One of the problems with encapsulation has been stability, especially enzyme stability. In many examples below, sorbitol has been substituted for enzymes due to their like densities and hydrophilic nature.

| Capsule formulation | |
|---|---|
| 2% Kraton® 1702 | 35% |
| Bowax® 800 | 20% |
| 70% Sorbitol aqueous solution | 45% |

2% Kraton® 1702 liquid gel was prepared by mixing 2 parts of Kraton® 1702 and 98 parts of mineral oil and heated to 76.7°C until it became an isotropic liquid gel. 35 parts of this prepared 2% Kraton® 1702 gel was mixed with 20 parts of Bowax® 800 at 60°C and followed by mixing in 45 parts of 70% sorbitol aqueous solution to form an emulsion. This emulsion was used to form capsules in an aqueous curing solution containing 1% Silwet® L77.

**Equipment used:**

Re-circulating encapsulation unit powered by a peristaltic pump. Lightening mixer was used to create movement in water bath so capsules didn't land on one another.

Results were capsules that were discrete in that they didn't stick together when they came in contact with one another.

EXAMPLE 3

Kraton® 1702 and mineral oil mixture form a continuous phase (liquid gel) at room temperature, which allows for uniformed mixing of components. An emulsion encapsulating enzymes was prepared at room temperature, thus avoiding the use of any heat in the process. Components were mixed together at room temperature using an overhead Lightening® mixer.

| Mineral oil | 235.2 g |
|---|---|
| Kraton® 1702 | 9.8 g |
| Properase® 1600L | 55.0 g |

The Kraton® /mineral oil mixture was transparent at room temperature. The enzyme in the sample of this emulsion was shown to have maintained activity. In the detergent composition, these capsules form a layer at the top of the composition.

EXAMPLE 4

Sodium Perborate is a good oxygen source for heavy duty liquids ("HDL"). It is well known, however, that boron ion may interact with PVA (polyvinyl alcohol) film, which results in the loss of the film solubility in water. This example demonstrates the incorporation of sodium perborate in a HDL, especially in a unit dose form.

The HDL composition that was used is summarized in the following table. The order of addition in the preparation of the HDL corresponded to the order on the list of raw materials in the table below.

| HDL Formula | | |
|---|---|---|
| Raw Material | % in formula | Supplier |
| Neodol® 1-5(alcohol ethoxylate, $C_9$-$C_{11}$, 5 ethylene oxide groups) | 26.62 | Shell Chemical |
| Monopropylene Glycol | 26.86 | Dow Chemical |
| Monoethanolamine | 7.56 | Dow Chemical |
| Oleic Acid | 13.10 | Uniquema |
| Fluorescent Dye | 0.20 | Ciba-Geigy |
| Softened Water | 3.27 | - |
| Linear Alkylbenze Sulfonic Acid | 20.72 | Stepan |
| MIscellaneous | 1.67 | Fragrance/Dy e |

Table continued

| HDL Formula | | |
|---|---|---|
| Raw Material | % in formula | Supplier |
| Total | 100.00 | |

10 g of sodium perborate ex. DuPont was dispersed in 40 g of petrolatum, Snow White® grade ex. Penreco. The petrolatum protected perborate from attacking the PVA film. C-120T grade, 76.2 $\mu$m thickness PVA film ex PVAXX was used for pouch making. The PVA film was folded and sealed three sides with a U-line sealer, KF-300H, at about 3.5 heat setting. The hydrophobic layer was filled first through the opening, followed by the filling of HDL. (50 grams). The opening was then sealed with a KF-300H sealer at the same setting.

One week later, the sodium perborate layer remained at the bottom of the pouch.

EXAMPLE 5

Sodium Nonanoyloxy Benzene Sulphonate is a peroxide precursor used in powder detergents, but its high solubility in water and reactivity to peroxide prevent its use in HDL. This example demonstrates the incorporation of Sodium Nonanoyloxy Benzene Sulphonate in a HDL pouch. 3g of Sodium Nonanoyloxy Benzene Sulphonate synthesized in the lab were dispersed in 50 g of Snow White® grade petrolatum. The dispersion was added to a pouch, followed by 50 g of HDL (HDL formula and pouch making and sealing procedure were as in Example 4). The pouch was then sealed. Within an hour, the dispersion floated to the top and remained at the top of the pouch. Again, Sodium Nonanoyloxy Benzene Sulphonate was protected by petrolatum.

EXAMPLE 6

It is beneficial to have both peroxide precursor and peroxide source protected in a HDL. 7.5 g of sodium perborate was dispersed in 35 g of Snow White® grade petrolatum, followed by the addition of 7.5 grams of Sodium Nonanoyloxy Benzene Sulphonate. The dispersion was added to a pouch, followed by 50 g of HDL. HDL formula and pouch making and sealing procedure were as in Example 4. One week later, the dispersion layer remained stable at the bottom of the pouch.

**Claims**

1. A water-soluble package for use in a single application comprising:

   (a) a fluid composition comprising water and a surfactant, for release on dissolution of the package, the composition comprising:
   (b) from about 0.1% to about 50%, by weight of the fluid composition, of a visually distinct layer composition comprising a hydrophobic ingredient, wherein the layer composition is in the form selected from the group consisting of capsules, an emulsion and a dispersion, and wherein the hydrophobic ingredient forms a hydrophobic continuous phase; and
   (c) a water-soluble body portion for containing the fluid composition and **characterised in that** the layer comprises a hydrophobic encapsulating material and an oil-soluble benefit agent, and the layer composition further comprises from about 0.01% to about 45%, by volume of the layer composition, of a discontinuous phase surrounded by the hydrophobic continuous phase, and the hydrophobic continuous phase is transparent or translucent.

2. The package of claim 1 wherein the layer composition further comprises an ingredient the density of which is higher than the density of the fluid composition.

3. The package of claim 1 wherein the layer composition further comprises from about 0.01 to about 50%, by weight of the layer composition, of an ingredient selected from the group consisting of a benefit agent, a colorant, and mixtures thereof.

4. The package of claim 1 wherein the discontinuous phase further comprises an ingredient selected from the group consisting of a benefit ingredient, a colorant, and mixtures thereof.

5. The package of claim 4 wherein the discontinuous phase is an aqueous solution of an enzyme.

6. The package of claim 5 wherein the layer composition is an emulsion.

7. The package of claim 1 wherein the continuous phase comprises a hydrocarbon oil and from about 0.1% to about 15%, by weight of the continuous phase, of a diblock co-polymer comprising at least one rigid block and at least one flexible block.

8. The package of claim 1 wherein the discontinuous phase comprises a colorant.

9. The package of claim 1 wherein the hydrophobic ingredient is selected from the group consisting of paraffin, oil, wax, petrolatum, a hydrophobic polymer, and mixtures thereof.

10. The package of claim 1 wherein the hydrophobic polymer is a thermoplastic polymer.

11. The package of claim 1 wherein the hydrophobic ingredient is a mixture of a thermoplastic polymer and hydrocarbon oil.

12. The package of claim 1 wherein the body portion is transparent.

13. The package of claim 1 wherein the fluid composition is a laundry detergent composition.

**Patentansprüche**

1. In Wasser lösliche Verpackung zur Verwendung in einer Einzelanwendung, umfassend:

   (a) eine Fluidzusammensetzung, umfassend Wasser und ein Tensid, zur Freisetzung bei Auflösung der Verpackung, wobei die Zusammensetzung umfasst:
   (b) etwa 0,1% bis etwa 50 Gew.-% der Fluidzusammensetzung einer visuell unterscheidbaren Schichtzusammensetzung, umfassend einen hydrophoben Bestandteil, wobei die Schichtzusammensetzung in der Form vorliegt, ausgewählt aus der Gruppe, bestehend aus Kapseln, einer Emulsion und einer Dispersion, und wobei der hydrophobe Bestandteil eine hydrophobe kontinuierliche Phase bildet; und
   (c) einen in Wasser löslichen Körperteil, der die Fluidzusammensetzung enthalten kann, und **dadurch gekennzeichnet, dass** die Schicht ein hydrophobes Einkapselungsmaterial und ein in Öl lösliches Vorteilsmittel umfasst, und die Schichtzusammensetzung weiterhin etwa 0,01 % bis etwa 45 %, auf das Volumen der Schichtzusammensetzung, einer diskontinuierlichen Phase, die durch die hydrophobe kontinuierliche Phase umgeben ist, umfasst und die hydrophobe kontinuierliche Phase transparent oder durchscheinend ist.

2. Verpackung nach Anspruch 1, worin die Schichtzusammensetzung weiterhin einen Bestandteil umfasst, dessen Dichte höher als die Dichte der Fluidzusammensetzung ist.

3. Verpackung nach Anspruch 1, worin die Schichtzusammensetzung weiterhin etwa 0,01 bis etwa 50%, auf das Gewicht der Schichtzusammensetzung, von einem Bestandteil, ausgewählt aus der Gruppe, bestehend aus einem Vorteilsmittel, einem Färbemittel und Gemischen davon, umfasst.

4. Verpackung nach Anspruch 1, worin die diskontinuierliche Phase weiterhin einen Bestandteil, ausgewählt aus der Gruppe, bestehend aus einem Vorteilsmittelbestandteil, einem Färbemittel und Gemischen davon, umfasst.

5. Verpackung nach Anspruch 4, worin die diskontinuierliche Phase eine wässrige Lösung eines Enzyms ist.

6. Verpackung nach Anspruch 5, worin die Schichtzusammensetzung eine Emulsion ist.

7. Verpackung nach Anspruch 1, worin die kontinuierliche Phase ein Kohlenwasserstofföl und etwa 0,1% bis etwa 15%, auf das Gewicht der kontinuierlichen Phase, eines Diblockcopolymers, umfassend mindestens einen starren Block und mindestens einen biegsamen Block, umfasst.

**8.** Verpackung nach Anspruch 1, worin die diskontinuierliche Phase ein Färbemittel umfasst.

**9.** Verpackung nach Anspruch 1, worin der hydrophobe Bestandteil aus der Gruppe, bestehend aus Paraffin, Öl, Wachs, Petrolatum, einem hydrophoben Polymer und Gemischen davon, ausgewählt ist.

**10.** Verpackung nach Anspruch 1, worin das hydrophobe Polymer ein thermoplastisches Polymer ist.

**11.** Verpackung nach Anspruch 1, worin der hydrophobe Bestandteil ein Gemisch von einem thermoplastischen Polymer und Kohlenwasserstofföl ist.

**12.** Verpackung nach Anspruch 1, worin der Körperteil durchscheinend ist.

**13.** Verpackung nach Anspruch 1, worin die Fluidzusammensetzung eine Wäschewaschmittelzusammensetzung ist.

**Revendications**

**1.** Emballage soluble dans l'eau pour une utilisation dans le cadre d'une application unique comprenant :

(a) une composition fluide comprenant de l'eau et un tensioactif destinée à être libérée au moment de la dissolution de l'emballage, la composition comprenant :
(b) d'environ 0,1 à environ 50 % en poids, sur la base de la composition fluide, d'une composition de couche distincte visuellement comprenant un ingrédient hydrophobe, dans laquelle la composition de couche est sous une forme sélectionnée dans le groupe constitué des capsules, d'une émulsion et d'une dispersion, et dans laquelle l'ingrédient hydrophobe forme une phase hydrophobe continue ; et
(c) une partie de corps soluble dans l'eau pour contenir la composition fluide, et **caractérisée en ce que** la couche comprend un matériau d'encapsulation hydrophobe et un agent bénéfique soluble dans l'huile et **en ce que** la composition de la couche comprend en outre d'environ 0,01 % à environ 45 %, en volume de la composition de couche, d'une phase discontinue entourée par la phase hydrophobe continue et **en ce que** la phase hydrophobe continue est transparente ou translucide.

**2.** Emballage selon la revendication 1, dans lequel la composition de couché comprend en outre un ingrédient dont la densité est supérieure à la densité de la composition fluide.

**3.** Emballage selon la revendication 1, dans lequel la composition de couche comprend en outre d'environ 0,01 % à environ 50 %, en poids de la composition de couche, d'un ingrédient sélectionné dans le groupe constitué d'un agent bénéfique, d'un colorant et de mélanges de ceux-ci.

**4.** Emballage selon la revendication 1, dans lequel la phase discontinue comprend en outre un ingrédient sélectionné dans le groupe constitué d'un ingrédient bénéfique, d'un colorant et de mélanges de ceux-ci.

**5.** Emballage selon la revendication 4, dans lequel la phase discontinue est une solution aqueuse d'une enzyme.

**6.** Emballage selon la revendication 5, dans lequel la composition de couche est une émulsion..

**7.** Emballage selon la revendication 1, dans lequel la phase continue comprend une huile hydrocarbonée et d'environ 0,1 % à environ 15 %, en poids de la phase continue, d'un copolymère dibloc comprenant au moins un bloc rigide et au moins un bloc flexible.

**8.** Emballage selon la revendication 1, dans lequel la phase discontinue comprend un colorant.

**9.** Emballage selon la revendication 1, dans lequel l'ingrédient hydrophobe est sélectionné dans le groupe constitué de la paraffine, de l'huile, de la cire, de la gelée de pétrole, d'un polymère hydrophobe et de mélanges de ceux-ci.

**10.** Emballage selon la revendication 1, dans lequel le polymère hydrophobe est un polymère thermoplastique.

**11.** Emballage selon la revendication 1, dans lequel l'ingrédient hydrophobe est un mélange d'un polymère thermoplastique et d'une huile hydrocarbonée.

**12.** Emballage selon la revendication 1, dans lequel la partie de corps est transparente.

**13.** Emballage selon la revendication 1, dans lequel la composition fluide est une composition détergente pour la lessive.